# FASCICULE DE BREVET EUROPEEN

(11) **EP 0 969 779 B1**
(45) Date de publication et mention de la délivrance du brevet: **01.06.2005**
(21) Numéro de dépôt: 98913811.0
(22) Date de dépôt: 03.03.1998
(51) Int. Cl.: A61F 2/06

(54) **DISPOSITIF FORMANT ENDOPROTHESE HELICODALE ET SON PROCEDE DE FABRICATION**
SCHRAUBENFÖRMIGE ENDOPROTHESE SOWIE HERSTELLUNGSVERFAHREN
DEVICE FORMING HELICAL STENT AND METHOD FOR MAKING SAME

(30) Priorité: 04.03.1997 FR 9702526
(43) Date de publication de la demande: 12.01.2000
(73) Titulaire: Glatt, Bernard, 75016 Paris (FR); Chevalier, Bernard, 95140 Groslay (FR); Royer, Thierry, 75018 Paris (FR); Guyon, Philippe, 92800 Puteaux (FR); Lecointe, Bruno, 92500 Rueil Malmaison (FR); Roy, Dominique, 78320 Le Mesnil Saint Denis (FR); Ascher, Gilles, 75116 Paris (FR)
(72) Inventeur: Glatt, Bernard, 75016 Paris (FR); Chevalier, Bernard, 95140 Groslay (FR); Royer, Thierry, 75018 Paris (FR); Guyon, Philippe, 92800 Puteaux (FR); Lecointe, Bruno, 92500 Rueil Malmaison (FR); Roy, Dominique, 78320 Le Mesnil Saint Denis (FR); Ascher, Gilles, 75116 Paris (FR)
(74) Mandataire: Portal, Gérard
(86) Numéro de dépôt international: PCT/FR1998/000409
(87) Numéro de publication internationale: WO 1998/038945

(56) Documents cités:
- EP-A- 0 540 290
- WO-A-95/31945
- WO-A-96/03092
- US-A- 5 314 472

## Description

L'invention concerne essentiellement un dispositif formant endoprothèse hélicoïdale ainsi que son procédé de fabrication.

On sait que les dispositifs formant endoprothèses aussi dénommés en anglais, ou en langage courant, "stents", permettent de traiter les rétrécissements de conduits divers de l'anatomie humaine tels qu'une artère et de les maintenir ouverts.

Il existe plusieurs types d'endoprothèses : tubulaires, réseaux, filamentaires, expansibles par ballonnets ou auto-expansibles.

Par exemple, le document US-A-5 578 149 ainsi que US-A-5 591 230 décrivent un dispositif formant endoprothèse formé par un élément filamentaire enroulé en hélicoïde en présentant des coudes définissant une structure en zig zag, permettant une expansion radiale importante avec un faible raccourcissement de la longueur du dispositif formant endoprothèse.

Le défaut de ces dispositifs formant endoprothèse est qu'ils ont une faible force d'étayage aux extrémités et un mauvais pouvoir de recouvrement aux extrémités.

Par ailleurs, on connaît aussi par le document US-A-4 553 545, divers modes de réalisation de dispositif formant endoprothèse formé par un élément filamentaire enroulé en hélicoïde. Il est présenté en référence à la figure 10 un risque de basculement ou d'écrasement ou de débobinage du dispositif formant endoprothèse, lors d'une expansion radiale, ce qui est aussi expliqué à la colonne 7, lignes 56 et suivantes. Pour remédier à cela, ce document propose de rigidifier soit toutes les spires par un élément de rigidification, voir figures 11 à 16. Il est proposé ensuite aux figures 22 et 23 un dispositif formant endoprothèse à structure en échelle, et notamment en référence à la figure 23, un dispositif formant endoprothèse formé par une hélicoïde à double hélice reliée par des éléments transversaux 82. Il est indiqué que cette structure permet d'améliorer la stabilité lors d'une expansion radiale (colonne 12, lignes 45 à 66).

Le défaut de ces dispositifs formant endoprothèse est qu'ils ont une faible force d'étayage aux extrémités et un mauvais pouvoir de recouvrement aux extrémités.

En outre, si lors de l'insertion du dispositif formant endoprothèse, on rencontre un obstacle, en raison d'une absence de tenue de l'hélicoïde aux extrémités, il peut se produire dans certains cas un tassement de l'hélicoïde.

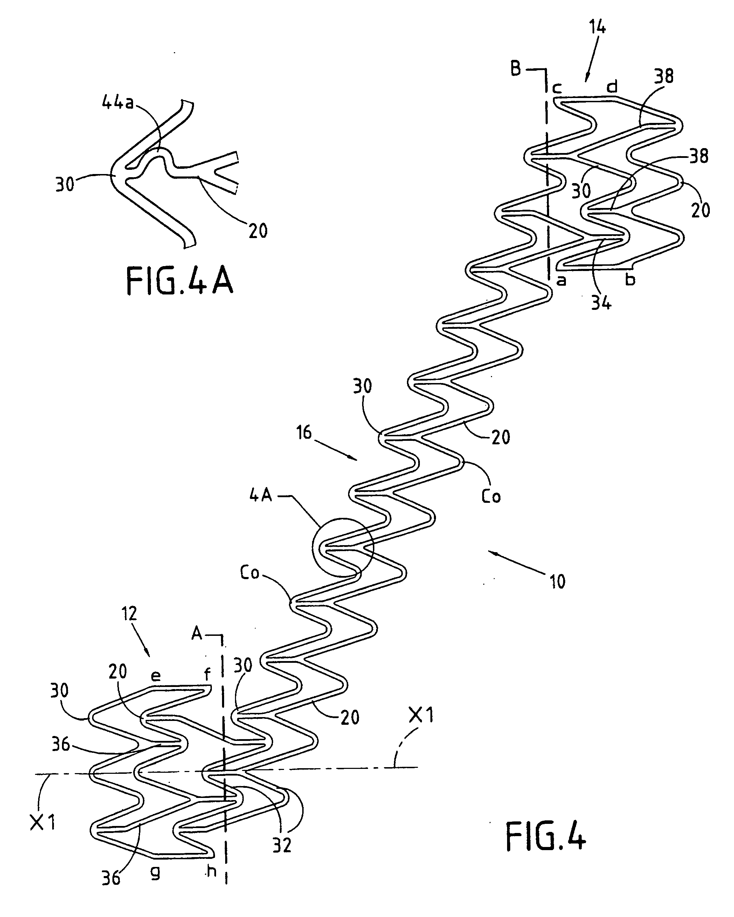

Il est encore connu par le document WO-A-96/03092, un dispositif formant endoprothèse constitué par un élément filamentaire enroulé de manière annulaire, constitué d'un certain nombre d'anneaux reliés entre eux par des éléments de liaison 22 coudés, les parties annulaires étant également coudées de manière à former une structure en zig zag. Ce dispositif présente une bonne force d'étayage, lors d'une expansion radiale. Ce dispositif présente une certaine flexibilité grâce à la présence des liens 22 comprenant des coudes 20, entre les éléments annulaires 11, mais cette flexibilité est essentiellement perdue après expansion et engendre des contraintes sur les conduits, et en particulier les artères. On observera que la structure décrite dans ce document fournit après déploiement un système en réseau ou cellulaire, comme cela est bien représenté à la figure 4, qui favorise l'étayage, mais limite la flexibilité et la conformabilité au conduit, ce qui constitue une exigence importante.

Les limites actuelles de ces dispositifs formant endoprothèse connus restent :
a) la rigidité de certaines endoprothèses, qui peut compromettre un accès à certaines lésions, dont l'amont est tortueux. Cette rigidité devient un inconvénient dans la mesure où il est de plus en plus recherché des dispositifs formant endoprothèse de plus grande longueur, tout en étant capable de réaliser des accès tortueux et pour lesquels il est recherché une plus grande flexibilité. Le dispositif formant endoprothèse décrit dans le document WO-A-96/03092 présente une flexibilité améliorée par rapport aux dispositifs antérieurs, mais qui est encore jugée insuffisante, notamment dans le cas de grandes longueurs, c'est-à-dire au-delà de 20 mm, ce qui est la tendance actuelle.
b) les contraintes engendrées sur l'artère en amont et en aval de l'endoprothèse résultant d'un phénomène d'étirement par rigidification du conduit après déploiement de l'endoprothèse. Ceci peut engendrer des complications pendant l'intervention ou des réactions tissulaires aboutissant à un rétrécissement récurrent du conduit précité. Ceci conduit à une conformabilité insuffisante au conduit, et notamment aux extrémités, engendrant des contraintes peu satisfaisantes auxdites extrémités, ce qui est en particulier le cas dans le document WO-A-96/03092.
c) la difficulté à refranchir l'endoprothèse lorsqu'elle est implantée à cheval sur une bifurcation et que la branche doit être également traitée. En particulier, il peut être nécessaire de passer un ballon ou une endoprothèse sur ballon à travers la structure de la première endoprothèse. Ceci est aussi en particulier le cas dans le document WO-A-96/03092.
d) Certaines endoprothèses manquent de force radiale dite d'étayage en particulier aux extrémités, ce qui peut être important pour étayer l'origine (ostium) d'un conduit tel qu'une artère, qui est en général très élastique. Ceci est en particulier le cas des dispositifs formant endoprothèse décrits dans les documents US-A-5 578 149, US-A-5 591 230, et US-A-4 553 545. La force d'étayage de l'ensemble de l'endoprothèse est également très importante lorsque les lésions exercent une forte résistance à la dilatation, comme cela est le cas des lésions calcifiées ou à fort recul élastique.
e) Certaines endoprothèses de type filamentaire se déforment longitudinalement lors d'un blocage de la progression pendant l'accès à une lésion en raison d'un manque de tenue de la structure. Ceci est en particulier le cas des dispositifs formant endoprothèse décrits dans les documents US-A-5 578 149, US-A-5 591 230, et US-A-4 553 545.

Ainsi, dans les dispositifs antérieurement connus, ceux-ci sont de conception soit entièrement hélicoïdale, soit entièrement annulaire. En outre, pour certaines endoprothèses de type annulaire, celles-ci peuvent être réalisées en réseau, comme dans le cas du document WO 96/03092.

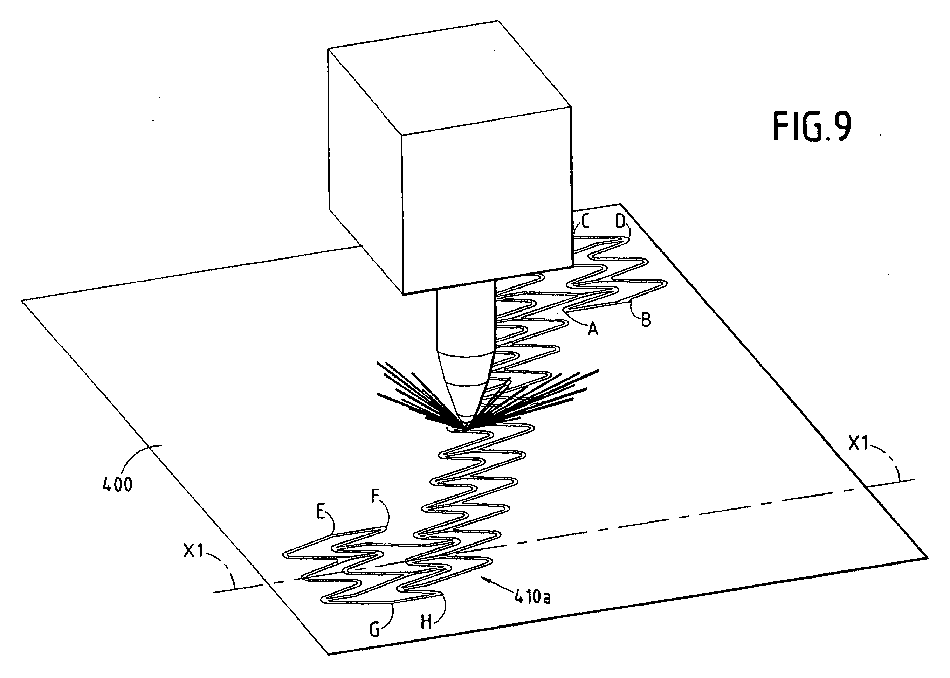

Encore, le document US-A-5 314 472 décrit à la figure 9 un dispositif comprenant un élément filamentaire dont la partie principale est enroulée de manière sinusoïdale et se poursuivant de manière continue également de manière sinusoïdale aux extrémités 19, 21 d'amplitude plus faible, de moitié à deux tiers de la hauteur de l'amplitude de la sinusoïde centrale et formant un angle avec la partie centrale (colonne 3, lignes 55 à 64, colonne 4, lignes 3 à 6 et colonne 5, lignes 51 à 58).

Ainsi, la présente invention a pour but principal de résoudre le nouveau problème technique consistant en la fourniture d'une solution, qui consiste en un nouveau dispositif formant endoprothèse, qui présente simultanément les propriétés suivantes :
a) une très bonne flexibilité,
b) une capacité de refranchissement latéral depuis l'intérieur de l'endoprothèse par une autre endoprothèse ou un dispositif à ballonnet,
c) une très bonne conformabilité dans le sens longitudinal, afin de ne pas modifier la courbure du conduit dans lequel l'endoprothèse doit être implantée, dans le sens longitudinal,
d) une très bonne force radiale afin de remplir complètement son rôle d'étayage du conduit dans lequel l'endoprothèse doit être implantée,
e) une très bonne capacité de refranchissement permettant un passage longitudinal par un autre dispositif tel qu'un ballon ou une autre endoprothèse, éventuellement également sur ballon,
f) une très bonne capacité de maintien en place en position déployée,
g) une très bonne capacité d'étayage et de couverture annulaire aux extrémités permettant une couverture complète sans débordement, ce qui est particulièrement important, notamment dans le domaine médical au niveau des ostiums.
h) une expansion essentiellement sans raccourcissement de la longueur de l'endoprothèse,
et ce même pour des endoprothèses de grandes longueurs, à savoir supérieures à environ 20 mm.

L'invention a encore pour but principal de fournir une solution au problème technique précédent selon une conception particulièrement simple, relativement peu coûteuse, utilisable à l'échelle industrielle et médicale.

Ainsi, selon un premier aspect, la présente invention fournit un dispositif formant endoprothèse tel que défini à la revendication 1. Ce dispositif permet de renforcer ainsi notamment le maintien de la structure en place ainsi que d'assurer une couverture complète sans débordement, en particulier au niveau d'un ostium.

Selon un mode de réalisation avantageux de l'invention, le dispositif comprend des parties d'extrémités définissant respectivement un bord d'extrémité proximale et un bord d'extrémité distale du dispositif, lesdites parties d'extrémités étant réalisées avec lesdits premier et deuxième éléments filamentaires enroulés sensiblement annulairement et reliés à ladite partie centrale composée du premier élément et du deuxième élément filamentaire précités en double hélice.

Selon un autre mode de réalisation avantageux de l'invention, ledit premier élément et ledit deuxième éléments filamentaire formant une structure en double hélice précitée sont coudés de manière à présenter une forme en zig zag, en présentant ici une forme en zig zag hélicoïdale.

Selon une variante de réalisation permettant un bon franchissement transversal, la partie centrale en double hélice comprend au moins 3 coudes définissant au moins 3 sommets par révolution de 360°.

Selon un mode de réalisation particulier de l'invention, les parties d'extrémités présentent une forme générale annulaire reliée directement ou par l'intermédiaire d'au moins un lien à la partie centrale en double hélice.

Selon un autre mode de réalisation particulier de l'invention, le bord proximal et le bord distal d'extrémités sont compris dans un plan de coupe circulaire, dont le centre coïncide sensiblement avec l'axe longitudinal défini par au moins la partie centrale précitée en double hélice.

Selon une variante de réalisation avantageuse, le bord proximal et le bord distal sont compris dans un plan de coupe circulaire sensiblement perpendiculaire à l'axe longitudinal défini par au moins la partie centrale précitée en double hélice.

Selon une autre variante de réalisation de l'invention, le bord proximal et le bord distal sont compris dans un plan de coupe circulaire formant un angle aigu avec l'axe longitudinal du dispositif, compris entre moins de 90° et au moins 60°, avantageusement entre moins de 90° et au moins 70°.

Selon un autre mode de réalisation avantageux de l'invention, lesdits premier élément filamentaire et deuxième élément filamentaire sont reliés entre eux par au moins quelques bras de liaison espacés le long de ladite partie centrale.

Selon une variante de réalisation, la double hélice étant composée de révolutions successives de 360°, au moins certaines révolutions comprennent au moins un bras de liaison entre le premier élément et le deuxième élément. Avantageusement, chaque révolution de 360° comprend au moins un bras de liaison.

Selon une autre variante de réalisation, chaque révolution comprend au moins deux bras de liaison.

Selon encore une autre variante de réalisation particulièrement avantageuse, certains bras de liaison ou chaque bras de liaison peut être lui-même coudé en présentant un ou plusieurs coudes de manière à améliorer la flexibilité permettant d'améliorer aussi la conformabilité au conduit.

Selon encore un autre mode de réalisation avantageux de l'invention, les bras de liaison précités présentent une dimension suffisante pour permettre de définir un espace suffisant entre le premier et le deuxième éléments hélicoïdaux précités, pour le passage d'un outil dans un conduit adjacent au conduit dans lequel le dispositif formant endoprothèse a été disposé.

Avantageusement, la longueur du bras de liaison est de l'ordre d'au moins 0,5 mm. Avantageusement, la longueur du bras de liaison sera comprise entre environ 0,5 mm et 5 mm.

Selon encore une autre variante de réalisation, la partie centrale comprend au moins un lien de renforcement r reliant en deux sommets CO de deux révolutions d'hélicoïde, qui se suivent. Avantageusement, ces liens de renforcement r peuvent être au nombre de un ou plusieurs par révolution, avantageusement en quinconce par rapport aux bras de liaison.

Selon un autre mode de réalisation de l'invention, la section droite d'au moins l'un ou des deux dits premier et deuxième éléments filamentaire précités est choisie parmi le groupe consistant d'une section droite circulaire, d'une section droite polygonale telle que carré, rectangulaire, pentagonale, hexagonale ou octogonale, d'une section droite polygonale à bord arrondi, telle que carré à bord arrondi ou rectangulaire à bord arrondi, ou d'une section droite ovoïde.

Selon un mode de réalisation particulier, la dimension de la section droite est directement liée à la dimension de la section droite du conduit dans lequel le dispositif formant endoprothèse doit être inséré. Par ailleurs, généralement, le diamètre dudit premier élément ou dudit deuxième élément, ou des deux, est compris entre environ un centième et environ un dixième du diamètre de la section droite définie par la partie d'extrémité annulaire du dispositif formant endoprothèse, dans sa forme initiale d'usinage prête à l'emploi.

Avantageusement, le diamètre du bras de liaison sera sensiblement égal du diamètre dudit élément filamentaire, avantageusement la forme du bras de liaison est aussi sensiblement identique à celle de l'élément filamentaire.

Selon un mode de réalisation, ledit premier élément de type filamentaire ou le deuxième élément filamentaire, ou les deux, définissent une hélicoïde présentant une inclinaison par rapport à l'axe longitudinal, au moins dans la partie centrale précitée, comprise entre environ 45° et environ 80°, avantageusement entre environ 50° et 70°, et encore mieux d'environ 60°.

Selon encore un autre mode de réalisation de l'invention, chaque coude de l'élément filamentaire précité présente un angle aigu défini par deux parties successives de l'élément filamentaire, compris entre environ 20° et environ 60°.

Selon encore un autre mode de réalisation avantageux de l'invention, le dispositif formant endoprothèse précité est réalisé monobloc, sans soudure, de son extrémité proximale à son extrémité distale, ce qui simplifie sa fabrication et élimine tout risque de corrosion et de rupture.

Dans le cadre de l'invention, on peut utiliser comme matériau constituant le dispositif formant endoprothèse tout matériau compatible avec une implantation dans le corps d'un mammifère, de préférence l'homme ou un animal. De tels matériaux sont généralement constitués par un matériau biocompatible, en particulier un polymère biodégradable ou un métal tel qu'un acier inox qualité médicale, un matériau à mémoire de forme, par exemple à base d'un alliage de nickel et de titane. On peut aussi utiliser des matériaux à faible mémoire élastique susceptible de subir une déformation plastique irréversible en exerçant une force mécanique radiale depuis l'intérieur vers l'extérieur, comme cela est bien connu à l'homme de l'art.

Selon un deuxième aspect, la présente invention fournit également un procédé de fabrication du dispositif formant endoprothèse précité, caractérisé en ce qu'il comprend la découpe d'un élément tubulaire à surface cylindrique pleine de manière à définir dans cet élément tubulaire ladite partie centrale en double hélice, ainsi que les parties d'extrémités précitées. Ainsi, il est possible de réaliser le dispositif formant endoprothèse monobloc et sans soudure d'une extrémité à l'autre.

Selon un mode de réalisation avantageux, cette découpe est réalisée de manière à définir le mode de réalisation précis prévu dans sa forme initiale à utiliser telle quelle.

Selon un troisième aspect, la présente invention fournit également un autre procédé de fabrication du dispositif formant endoprothèse précité caractérisé en ce qu'on réalise une découpe d'un élément plan ou plaque de manière à définir la partie centrale précitée, destinée à former une double hélice par enroulement sur elle-même autour d'un mandrin de forme appropriée, de préférence à section droite circulaire, ladite découpe réalisant aussi les parties d'extrémité précitées. Ainsi, il est possible de réaliser l'endoprothèse monobloc, mais une soudure à chaque extrémité est nécessaire après l'enroulement.

Ces découpes sont réalisées à l'aide de procédés et de dispositifs bien connus à l'homme de l'art. L'homme de l'art connaît par exemple des techniques d'usinage par laser, notamment de type YAG ou CO₂, ou autre, par électroérosion, ou par une combinaison des deux ; la technique d'électroérosion est aussi connue en anglais par "etching". On comprend que lorsque l'on part d'un tube, la technique par rayon laser consiste à attaquer la surface externe du tube par le faisceau laser de manière à définir la structure recherchée comme ici la structure en double hélice dans la partie centrale.

D'autre part, lorsque l'on utilise une plaque mince, par exemple rectangulaire, le rayon laser usine dans cette plaque la structure de l'invention comportant dans sa partie centrale la double hélice. Ensuite, il suffira de rouler la structure obtenue sur un mandrin cylindrique et enfin de souder les bords de chacune des extrémités.

On peut aussi partant soit du tube, soit de la plaque mince, utiliser la technique d'électroérosion ou "etching" précitée.

Selon encore un quatrième aspect, la présente invention fournit un autre procédé de fabrication du dispositif formant endoprothèse précité caractérisé en ce qu'on réalise une découpe d'un élément plan ou plaque, ou d'un tube, à l'aide d'un dispositif de projection d'un liquide sous pression, en particulier de l'eau, de type Karcher®.

Selon un cinquième aspect, la présente invention couvre aussi un procédé de fabrication selon lequel on fabrique tout d'abord ledit premier élément filamentaire précité de forme hélicoïdale et un deuxième élément indépendant filamentaire de forme hélicoïdale, puis on relie lesdits premier élément et deuxième élément selon un pas déterminé, à des parties d'extrémités définissant un bord proximal et un bord distal du type précédemment décrit dans le cadre de l'invention avec éventuellement la présence de bras de liaison et/ou de liens comme précédemment décrit.

Grâce à l'invention, on résout les problèmes techniques précédemment énoncés d'une manière simple, relativement peu coûteuse, utilisable à l'échelle industrielle et médicale.

Dans le cadre de l'invention, on observe que par une combinaison d'extrémité(s) essentiellement annulaire(s) et de partie centrale de type hélicoïdal, on aboutit de manière inattendue pour un homme de l'art à l'obtention simultanée de l'ensemble des exigences précédemment énoncées, à savoir une très bonne flexibilité, une très bonne capacité de refranchissement latéral depuis l'intérieur, une très bonne conformabilité dans le sens longitudinal, une très bonne force radiale, en particulier aux extrémités, une très bonne capacité d'étayage et de couverture annulaire aux extrémités permettant une couverture complète sans débordement, une expansion essentiellement sans raccourcissement de la longueur de l'endoprothèse, une très bonne capacité de refranchissement permettant un passage longitudinal par un autre dispositif, tel qu'un ballon ou une autre endoprothèse, éventuellement également sur-ballon, une très bonne capacité de maintien en place en position déployée combinée avec une très bonne capacité d'étayage, même dans la partie centrale de type hélicoïdal, et ce même pour des dispositifs formant endoprothèse de grandes longueurs, c'est-à-dire de longueurs supérieures à environ 20 mm, et pour des dimensions pouvant être supérieures à 50 mm.

En outre, l'invention, par la combinaison d'une partie centrale hélicoïdale, de préférence en zig zag, avec des extrémités sensiblement annulaires, on permet un sertissage aisé sur un dispositif de mise en place, notamment de type à ballonnet, ce que ne permettaient pas les dispositifs antérieurs de type hélicoïdal, tels que décrits dans le document US-A-4 553 545.

L'invention est en outre applicable à des dispositifs auto-expansibles ou non.

D'autres buts, caractéristiques et avantages de l'invention apparaîtront clairement à la lumière de la description explicative, qui va suivre, faite en référence aux dessins annexés, représentant plusieurs modes de réalisation actuellement préférés de l'invention, donnés simplement à titre d'illustration, et qui ne sauraient donc en aucune façon limiter la portée de l'invention.

Dans les figures :

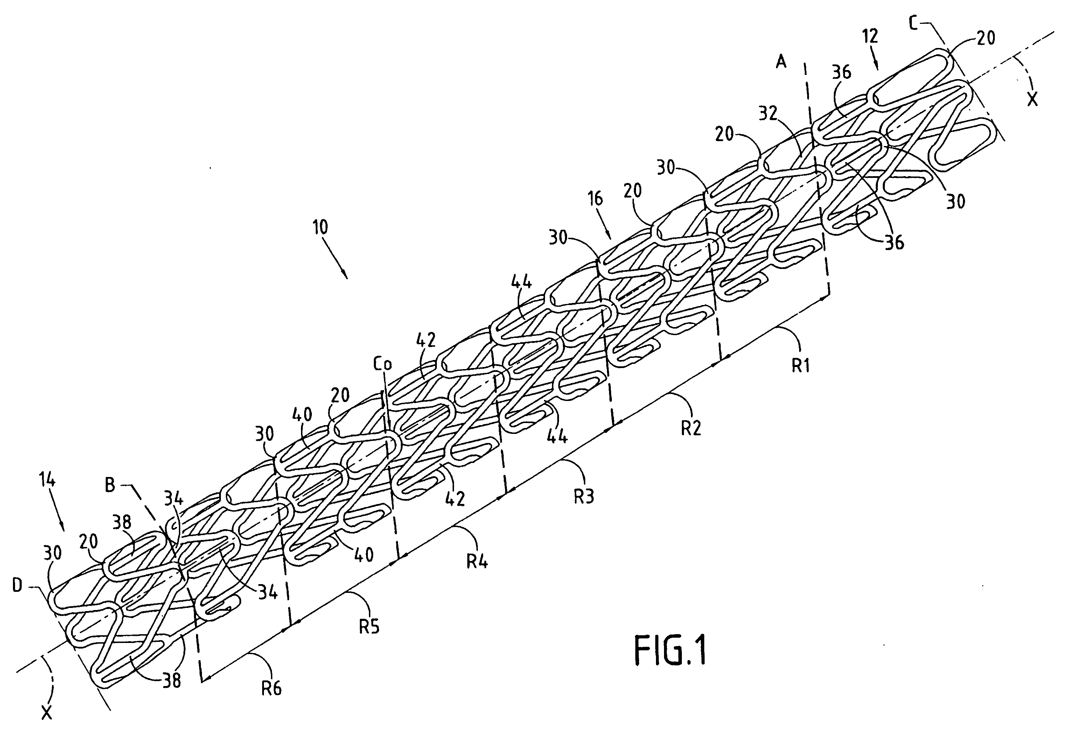

- la figure 1 représente un premier mode de réalisation actuellement préféré d'un dispositif formant endoprothèse, selon la présente invention ;

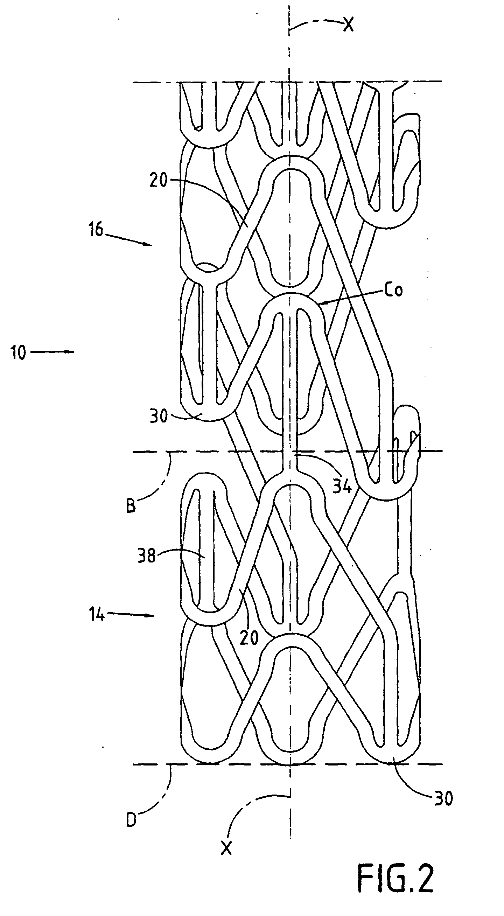

- la figure 2 représente une vue de détail de l'extrémité, telle que l'extrémité distale du dispositif de la figure 1 ;

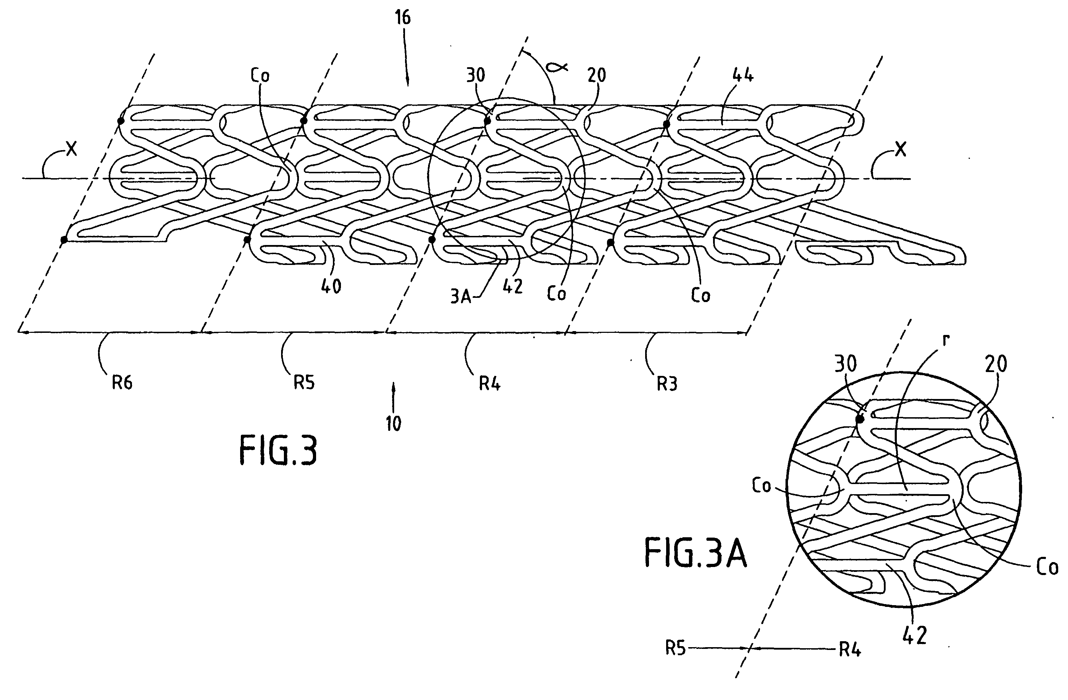

- la figure 3 représente de manière agrandie la partie centrale du mode de réalisation de la figure 1 ;
- la figure 3a représente une modification de réalisation de la partie centrale du mode de réalisation de la figure 1, dans laquelle sont présents des liens de renforcement r ;
- la figure 4 représente un dispositif formant endoprothèse selon l'invention réalisé plan, qui par un enroulement cylindrique permet d'obtenir le mode de réalisation des figures 1 à 3 ;
- la figure 4a représente une vue de détail du dispositif de la figure 4 au niveau d'un bras de liaison permettant de montrer une variante de réalisation selon laquelle le bras de liaison peut être coudé ;

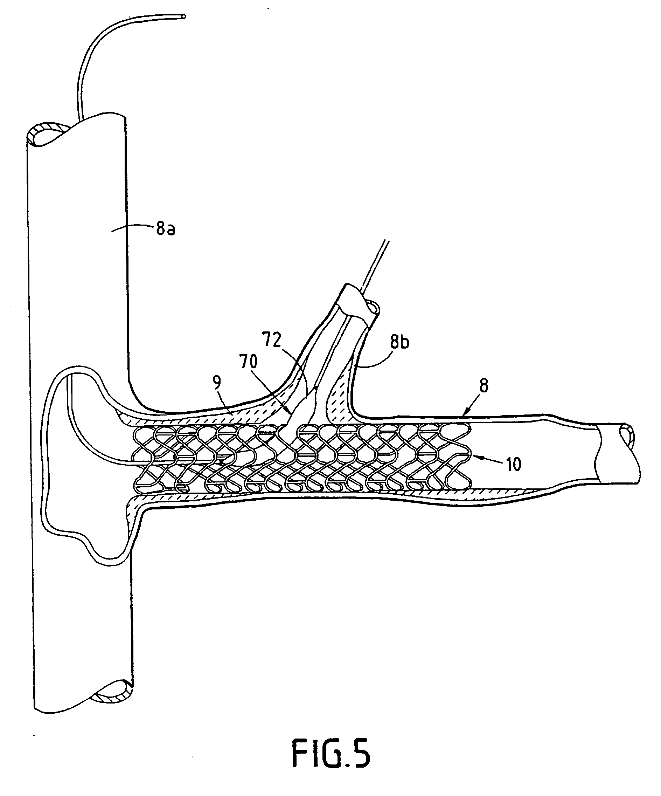

- la figure 5 représente le dispositif des figures 1 à 3 en position dans un conduit tel qu'une artère ;

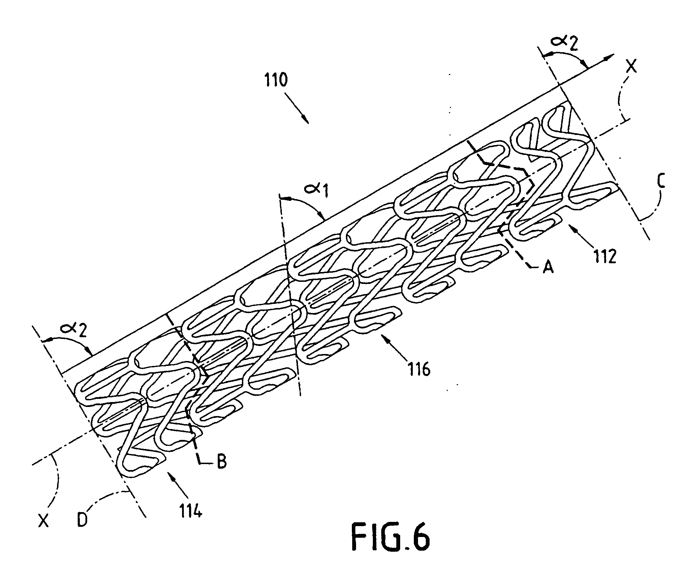

- la figure 6 représente un deuxième mode de réalisation selon l'invention avec un pas variable de l'hélicoïde ;

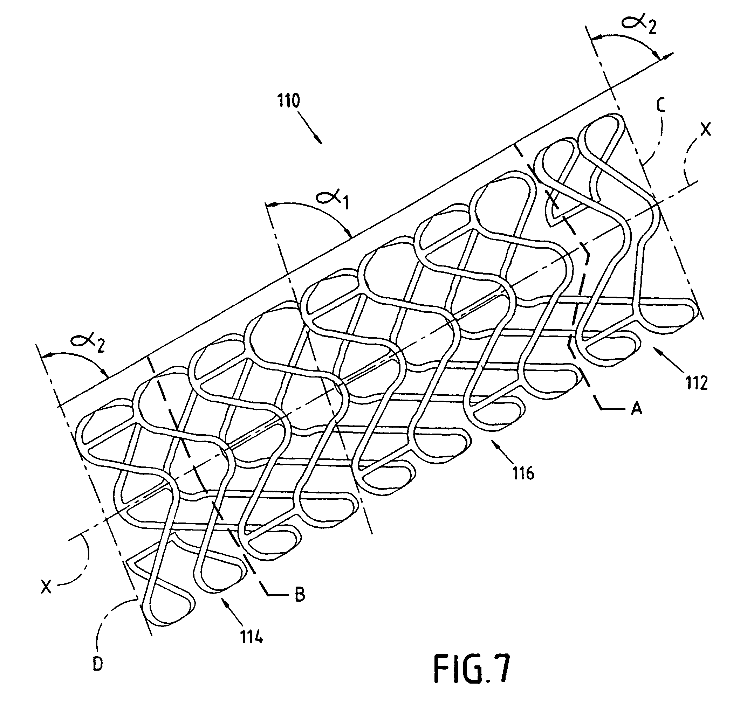

- la figure 7 représente le dispositif de la figure 6 à l'état déployé ;

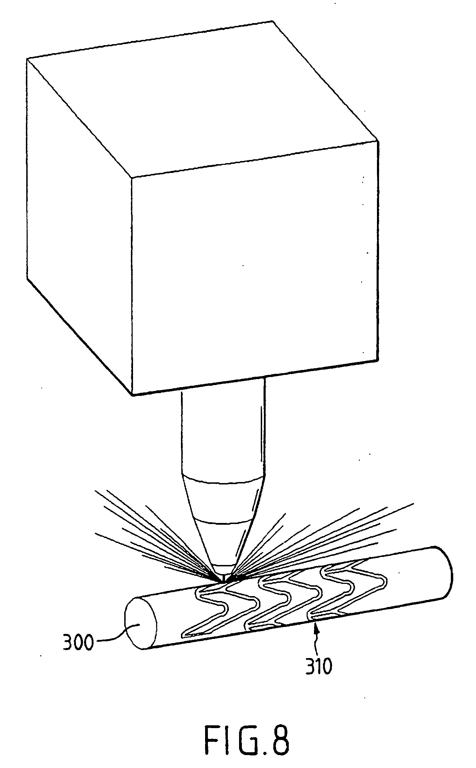

- la figure 8 représente un procédé de fabrication à partir d'un tube creux à paroi cylindrique pleine par découpe ; et
- la figure 9 représente un autre mode de fabrication du dispositif selon l'invention par découpe dans un élément plan ou plaque.

La figure 1 représente un premier mode de réalisation actuellement préféré d'un dispositif formant endoprothèse, selon la présente invention, représenté par le numéro de référence général 10. Ce dispositif formant endoprothèse comprend une partie d'extrémité proximale 12 et une partie d'extrémité distale 14 reliées entre elles par une partie centrale 16.

Ce dispositif formant endoprothèse 10 comprend un premier élément 20 de type filamentaire enroulé hélicoïdalement au moins dans la partie centrale 16.

Selon l'invention, ce dispositif est caractérisé en ce qu'il comprend un deuxième élément 30 de type filamentaire enroulé hélicoïdalement au moins dans la partie centrale 16, lesdits premier et deuxième éléments définissant ainsi une structure en double hélice, comme cela est bien visible aux figures 1 à 8.

Egalement, dans le cadre de l'invention, au moins une, de préférence les deux, partie(s) d'extrémité(s) 12 et 14 sont réalisées avec au moins un dit élément filamentaire 20 ou 30, de préférence les deux, enroulés sensiblement annulairement. Cette structure permet de renforcer notamment le maintien de la structure en place et de fournir une couverture complète sans débordement. Il est à noter à la figure 1 que les droites en pointillés, repérées A et B, permettent de symboliser la séparation entre les parties d'extrémités 12 et 14, et la partie centrale 16.

Selon un mode de réalisation avantageux de l'invention, le dispositif 10 comprend des parties d'extrémités 12 et 14 définissant respectivement un bord d'extrémité proximale repéré symboliquement par la droite C et un bord d'extrémité distale repéré symboliquement par la droite D. Les parties d'extrémités 12 et 14 peuvent être reliées à la partie centrale 16 directement ou par l'intermédiaire d'au moins un lien tel que le lien 32 pour la partie d'extrémité 12 et 34 pour la partie d'extrémité 14.

Il est à noter que la partie d'extrémité 12 et la partie d'extrémité 14 peuvent être réalisées différentes, comme cela apparaît à la figure 1. En outre, chaque partie d'extrémité 12 ou 14, peut être réalisée à l'aide de deux éléments filamentaires enroulés sensiblement annulairement et reliés entre eux par des bras de liaison particuliers, tels que 36, 38, respectivement.

Par ailleurs, en général, les bras de liaison successifs sont de longueurs différentes, comme cela se voit aussi à la figure 1, en particulier dans la partie centrale 16, et sera expliqué plus en détail relativement aux autres figures.

Selon un autre mode de réalisation particulier de l'invention, le bord proximal C et le bord distal D d'extrémités sont compris dans un plan de coupe sensiblement circulaire, dont le centre coïncide sensiblement avec l'axe longitudinal défini par au moins la partie centrale 16 en double hélice.

Selon une autre variante de réalisation avantageuse, le bord proximal C et le bord distal D sont compris dans un plan de coupe circulaire sensiblement perpendiculaire à l'axe longitudinal X-X défini par au moins la partie centrale 16 précitée en double hélice, comme représenté à la figure 1.

Selon une autre variante de réalisation de l'invention, le bord proximal C et le bord distal D sont compris dans un plan de coupe circulaire formant un angle aigu avec l'axe longitudinal du dispositif, compris entre moins de 90° et au moins 60°, de préférence entre moins de 90° et au moins 70°, comme cela est défini par le mode de réalisation de la figure 6, qui sera expliqué plus loin.

Il est à noter que pour permettre une transition entre les parties d'extrémités 12 et 14 et la partie centrale 16, la fin des parties d'extrémités présente une forme particulière assurant la transition entre les parties d'extrémités sensiblement annulaires et la partie centrale sensiblement hélicoïdale, ce qui est aussi visible à la figure 1.

Selon un autre mode de réalisation avantageux de l'invention, le premier élément 20 et le deuxième élément 30 de type filamentaire forment une structure en double hélice et sont coudés de manière à présenter une forme en zig zag, telle que représentée à la figure 1, en présentant ici une forme en zig zag hélicoïdale.

Selon une variante de réalisation permettant un bon franchissement transversal, la partie centrale 16 en double hélice comprend au moins 3 coudes CO définissant au moins 3 sommets par révolution de 360°.

Dans la partie centrale 16, on observera que le premier élément 20 de type filamentaire et le deuxième élément 30 de type filamentaire sont reliés entre eux par au moins quelques bras de liaison tels que 40, 42, 44 espacés le long de ladite partie centrale 16.

Selon une variante de réalisation, la double hélice est composée de révolutions successives de 360°, référencées ici R₁ à R₆ par exemple, au moins certaines révolutions comprennent au moins un bras de liaison tel que 40, 42, 44 entre le premier élément et le deuxième élément. Avantageusement, chaque révolution à 360° comprend au moins un bras de liaison, tel que 40, 42, 44.

Selon une autre variante de réalisation, chaque révolution comprend au moins deux bras de liaison, tels que 40, 42, 44. Dans l'exemple représenté, chaque révolution comprend trois bras de liaison.

Selon une autre variante de réalisation avantageuse, au moins certains bras de liaison, et en particulier chaque bras de liaison, au moins dans la partie centrale 16 peut présenter au moins un coude, comme représenté à la figure 4, ce qui améliore la flexibilité et la conformabilité du dispositif, ce qui est particulièrement important dans le cadre d'endoprothèse de grandes longueurs, c'est-à-dire d'une longueur d'au moins 20 mm, ce qui est actuellement de plus en plus recherché.

Selon encore un autre mode de réalisation avantageux de l'invention, les bras de liaison précités, tels que 32, 34, 36, 40, 42, 44 présentent une dimension suffisante pour permettre de définir un espace suffisant entre le premier et le deuxième éléments 20,30 hélicoïdaux précités pour le passage d'un outil dans un conduit adjacent ou conduit dans lequel le dispositif formant endoprothèse a été disposé. Avantageusement, la longueur des bras de liaison tels que 40, 42, 44 dans la partie centrale 16 sera identique. La longueur du bras de liaison peut être variable entre de larges limites. Avantageusement, la longueur du bras de liaison sera d'au moins 0,5 mm.

Selon une variante de réalisation actuellement préférée, la longueur de bras de liaison sera comprise entre environ 0,5 mm et environ 5 mm. En pratique, la dimension sera certainement médiane entre ces valeurs.

Selon un autre mode de réalisation avantageux de l'invention, les parties de l'élément filamentaire 20 ou de l'élément filamentaire 30, adjacentes à un coude donné et donc le définissant, seront de longueurs différentes dans la partie centrale 16 afin de permettre de définir au moins en partie le pas hélicoïdal recherché. On comprend qu'au moins une partie de ce pas hélicoïdal est défini par l'inclinaison de chaque élément filamentaire dans une révolution, ainsi que la dimension du bras de liaison, tels que 40, 42, 44, comme cela est bien compréhensible à un homme de l'art à partir de la structure géométrique, telle que représentée à la figure 1, en considérant les révolutions notées telles que R₁ à R₆.

Selon un autre mode de réalisation de l'invention, la section droite d'au moins l'un ou des deux premier et deuxième éléments de type filamentaire 20, 30 précités est choisie parmi le groupe consistant en une section droite circulaire, une section droite polygonale, telle que carré, rectangulaire, pentagonale, hexagonale ou octogonale, d'une section droite polygonale à bord arrondi, telle que carré à bord arrondi ou rectangulaire à bord arrondi, ou d'une section droite ovoïde.

Selon un autre mode de réalisation particulier, la dimension de la section droite de l'endoprothèse est directement liée à la dimension de la section droite du conduit dans lequel le dispositif formant endoprothèse doit être inséré.

Par ailleurs, généralement, le diamètre dudit premier élément ou dudit deuxième élément, ou des deux, est compris entre environ un centième et environ un dixième du diamètre de la section droite du dispositif formant endoprothèse 10 dans sa forme initiale d'usinage prête à l'emploi, telle que représentée à la figure 1. Avantageusement, le diamètre du bras de liaison sera sensiblement voisin du diamètre de l'élément de type filamentaire, comme représenté à la figure 1.

Selon un mode de réalisation, le premier élément de type filamentaire 20 ou deuxième élément de type filamentaire 30, ou les deux, définissent une hélicoïde présentant une inclinaison par rapport à l'axe longitudinal, au moins de la partie 16, défini par l'angle α, figure 3, comprise entre environ 45° et environ 80°, avantageusement entre environ 50° et 70°, de manière actuellement préférée d'environ 60°.

Selon encore un autre mode de réalisation de l'invention, chaque coude référencé CO de l'élément type filamentaire 20 ou 30 présente un angle aigu défini par deux parties successives de l'élément filamentaire 20 ou 30, compris entre environ 20° et environ 60°. Comme il a été dit précédemment, ces parties successives sont de longueurs différentes, au moins dans la partie centrale 16.

On peut prévoir dans certains cas que tous les coudes ne soient pas identiques, ce qui peut être le cas entre les parties d'extrémités 12 et 14 et la partie centrale 16 et notamment dans la partie de transition entre ces parties d'extrémités 12, 14 et la partie centrale 16.

Naturellement, on pourra utiliser comme matériau constituant le dispositif formant endoprothèse tout matériau compatible avec une implantation dans le corps d'un mammifère, de préférence l'homme ou un animal. De tels matériaux sont généralement constitués par un métal biocompatible, en particulier un acier inox qualité médicale, matériau à mémoire de forme, par exemple à base d'un alliage de nickel et de titane.

La figure 2 représente une vue de détail d'une extrémité, telle que l'extrémité distale 14, et la zone de transition au niveau de la droite B avec la partie centrale 16. On observe que la partie d'extrémité 14 comprend un premier élément filamentaire, ici de préférence constitué par le même élément filamentaire que l'élément 20, ici enroulé en partie annulairement, pour réaliser la zone de transition avec la partie centrale 16, de manière à définir le bord distal D contenu dans un plan de coupe circulaire sensiblement perpendiculaire à l'axe longitudinal X-X du dispositif formant endoprothèse 10. La partie d'extrémité 14 comprend aussi un deuxième élément filamentaire, qui est identique au deuxième élément filamentaire 30, qui est enroulé dans la partie d'extrémité 14 de manière annulaire.

A la figure 3, on a représenté de manière agrandie la partie centrale 16 pour mieux visualiser les révolutions, ici R₃ à R₆, ainsi que l'angle α d'inclinaison de l'hélicoïde. Dans l'exemple représenté aux figures 1 et 3, cet angle d'inclinaison est d'environ 62°.

Le dispositif formant endoprothèse représenté aux figures 1 à 3 peut être obtenu à partir d'une plaque par usinage à plat, qui sera décrit en référence avec la figure 9, et qui donne une forme développée plane représentée à la figure 4, avant roulage et soudage.

En référence à la figure 3a, on a représenté un mode de réalisation modifié de celui de la figure 3 de la partie centrale du mode de réalisation de la figure 1, selon lequel on prévoit au moins un lien de renforcement r reliant deux sommets CO de deux révolutions d'hélicoïde, qui se suivent, par exemple ici entre la révolution R₄ et la révolution R₅. La présence d'un tel lien de renforcement r peut être réalisée seulement sur certaines révolutions d'hélicoïde. La présence d'un tel lien de renforcement r peut être une solution avantageuse pour renforcer la tenue de l'ensemble et la force d'étayage.

Ces liens r peuvent être au nombre de un ou de plusieurs par révolution, avantageusement disposés en quinconce par rapport aux bras de liaison 40, 42 ou 44.

A la figure 4, on voit clairement les parties d'extrémités proximale 12 et distale 14 séparées sensiblement par les droites A et B de la partie centrale 16. On a utilisé les mêmes références qu'à la figure 1 pour montrer les parties identiques. De ce fait, la description de l'élément plan se comprend aisément à partir de la description, qui a été réalisée pour la forme enroulée cylindrique des figures 1 à 3.

A la figure 4a, on a représenté une vue détail d'une variante de réalisation d'un bras de liaison, selon laquelle le bras de liaison, ici 44, présente au moins un coude 44a, qui peut être simple, ou même double, en présentant dans ce cas une forme en double S, de préférence dans le même plan que les éléments 20 et 30, comme cela est bien compréhensible à un homme de l'art. Cette variante de réalisation permet d'améliorer la flexibilité, la conformabilité et la facilité du déploiement du dispositif formant endoprothèse, lorsqu'un nombre suffisant de bras de liaison présente ce coudage.

La figure 5 représente le dispositif formant endoprothèse de l'invention 10 selon la présente invention en position dans un conduit 8, tel qu'une artère, qui comporte une zone à traiter 9, telle qu'une sténose non occlusive ou occlusive, qui a été transpercée. A la figure 5, il est représenté le dispositif selon l'invention 10, sous forme déployée. Dans le cadre de l'invention, l'expansion radiale ne raccourcit sensiblement pas la longueur du dispositif formant endoprothèse 10, et d'autre part maintient les bords d'extrémités dans un plan de coupe sensiblement circulaire, sensiblement perpendiculaire à l'axe longitudinal X-X du dispositif formant endoprothèse, qui coïncide ici également sensiblement avec l'axe longitudinal du conduit dans lequel le dispositif est inséré.

On observe encore à la figure 5 que le conduit 8, tel qu'une artère, est ici représenté débutant à l'ostium, en dérivation d'un conduit plus grand, ici une artère 8a, ainsi que vers sa partie centrale d'un conduit colatéral 8b, tel qu'une artère plus étroite. On observe ici que la sténose 9 débute à l'ostium et s'étend au-delà du conduit colatéral 8b, qui est aussi initialement obturé par la sténose 9. On observe que grâce à l'invention, il est possible de réaliser un étayage du conduit 8 au niveau de l'ostium, sans débordement, grâce à l'extrémité annulaire. En outre, il est possible de réaliser un franchissement aisé du dispositif latéralement pour permettre le passage d'un autre dispositif 70, par exemple ici à ballonnet 72, pour traiter la sténose au niveau du conduit colatéral 8b.

En référence à la figure 6, on a représenté un deuxième mode de réalisation d'un dispositif formant endoprothèse selon l'invention, à l'état initial non déployé, représenté ici par le numéro de référence général 110. De ce fait, les références sont augmentées de 100 dans ce deuxième mode de réalisation par rapport au mode de réalisation des figures 1 à 3. Ainsi, ce dispositif comprend une première partie d'extrémité 112 et une deuxième partie d'extrémité 114 et une partie centrale 116. Dans ce deuxième mode de réalisation, le pas de l'hélicoïde varie progressivement depuis la partie centrale 116, en formant un angle d'inclinaison α1, jusqu'aux parties d'extrémités 112 et 114, en formant alors un angle d'inclinaison α2, pour aboutir au fait que les parties d'extrémités 112 et 114 présentent sensiblement une forme annulaire. On a représenté, à la partie d'extrémité 112, l'angle d'inclinaison α2 résiduel par rapport à l'axe longitudinal X-X du dispositif formant endoprothèse. Cet angle résiduel α2 sera généralement compris entre moins de 90° et au moins 60°, de préférence entre moins de 90° et au moins 70°. Dans le mode de réalisation représenté à la figure 6, cet angle d'inclinaison défini par rapport à l'axe longitudinal X-X est de l'ordre de 85°, ce qui fait une inclinaison d'environ 5° par rapport à la perpendiculaire à l'axe longitudinal.

On notera que ce mode de réalisation permet toujours d'aboutir au résultat de l'invention, à savoir éviter un débordement aux extrémités, car le déploiement contribue à augmenter l'angle d'inclinaison par rapport à l'axe longitudinal X-X, c'est-à-dire à le réduire par rapport à l'axe perpendiculaire à l'axe longitudinal, ce qui peut s'observer pour un homme de l'art par la forme déployée de la figure 7 du dispositif de la figure 6.

La figure 8 représente un procédé de fabrication, pas en harmonie avec l'invention, à partir d'un tube 300 creux à paroi cylindrique pleine, par découpe, qui peut être réalisée selon plusieurs procédés, pour aboutir un dispositif formant endoprothèse, selon l'invention, représenté par le numéro de référence général 310. Un premier procédé consiste à réaliser une découpe laser, un deuxième procédé consiste à réaliser une électroérosion et un troisième procédé consiste à réaliser une érosion par jet d'un liquide sous pression, en particulier par jet d'eau sous pression du type Karcher®.

La figure 9 représente une autre méthode de fabrication, pas en harmonie avec l'invention, qui consiste à partir d'un élément plan ou plaque 400, dans lequel on réalise la découpe par les moyens de découpe, qui viennent d'être décrits pour la figure 8, à savoir découpe laser, découpe par électroérosion ou découpe par érosion par un liquide sous pression, pour obtenir un dispositif formant endoprothèse représenté par le numéro de référence général 410a. On obtient, dans le cas de la figure 9, un élément 410a du type de celui décrit à la figure 4, et qui doit être enroulé sur lui-même selon l'axe d'enroulement X1-X1, et qui permet ensuite de définir l'axe longitudinal X-X du dispositif formant endoprothèse, qui a aussi été représenté à la figure 4.

Dans ce mode de réalisation à partir d'un élément plan, on comprend que l'enroulement permet d'amener les bords AB sur CD et EF sur GH, qui sont ensuite soudés pour éviter naturellement une réouverture du système. Ce mode de réalisation apparaît très pratique pour une découpe à partir d'une plaque, mais nécessite un enroulement et des soudures, ce qui doit être évité dans certains cas d'application. De ce fait, pour d'autres cas, on préférera une découpe à partir d'un tube, comme décrit à la figure 8.

On comprend que l'invention donne lieu à divers modes de réalisation et comprend tous les moyens constituant des équivalents techniques des moyens décrits et représentés. Les figures 1 à 9 font partie intégrante de la présente invention et donc de la présente description.

L'invention couvre aussi toute caractéristique qui apparaît nouvelle à partir de la description et des dessins eux-mêmes, par rapport à l'art antérieur quelconque, et ce, à titre de moyen général.

On comprend également que l'on peut combiner les divers modes de réalisation ou de fabrication entre eux. Par exemple, il est possible de réaliser la partie centrale 16 à partir d'un élément plan ou plaque, et les parties d'extrémités à partir d'élément tubulaire creux et de les combiner ensemble au moment de l'assemblage.

Dans le cadre de l'invention, les bras de liaison 40, 42, 44 avantageusement relient une partie convexe d'un sommet à une partie concave d'un autre sommet, comme représenté aux figures annexées. Généralement, dans les modes de réalisation actuellement préférés, les bras de liaison sont prévus sur un même élément filamentaire un coude sur deux, mais on comprend qu'on peut modifier cette disposition à volonté, selon le but recherché.

## Revendications

1. Dispositif formant endoprothèse comprenant un premier élément (20) filamentaire enroulé hélicoïdalement au moins dans sa partie centrale, ainsi qu'une partie d'extrémité proximale et une partie d'extrémité distale, dont au moins une partie d'extrémité est réalisée avec ledit élément enroulé sensiblement annulairement, **caractérisé en ce qu'**il comprend un deuxième élément (30) filamentaire enroulé hélicoïdalement au moins dans sa partie centrale, lesdits premier et deuxième éléments définissant ainsi une structure en double hélice ; et **en ce que** ledit premier élément (20) filamentaire et ledit deuxième élément (30) filamentaire sont reliés entre eux par au moins quelques bras de liaison (40, 42, 44) espacés le long de ladite partie centrale (16).

2. Dispositif selon la revendication 1, **caractérisé en ce que** ledit premier élément (20) et ledit deuxième élément (30) filamentaire formant une structure en double hélice précitée sont coudés de manière à présenter une forme en zig zag, en présentant ici une forme en zig zag hélicoïdale.

3. Dispositif selon l'une des revendications précédentes, **caractérisé en ce qu'**il comprend des parties d'extrémités (12, 14) définissant respectivement un bord d'extrémité proximale (C) et un bord d'extrémité distale (D) du dispositif, lesdites parties d'extrémités (12, 14) étant réalisées avec lesdits premier et deuxième éléments filamentaires enroulés sensiblement annulairement et reliés à ladite partie centrale (16) composée du premier élément (20) et du deuxième élément (30) filamentaire précités en double hélice.

4. Dispositif selon l'une des revendications 1 à 3, **caractérisé en ce que** les parties d'extrémités (12, 14) présentent une forme générale annulaire reliée directement ou par l'intermédiaire d'un lien (32, 34) à la partie centrale (16) en double hélice.

5. Dispositif selon l'une des revendications 1 à 4, **caractérisé en ce que** le bord proximal (C) et le bord distal (D) d'extrémités sont compris dans un plan de coupe circulaire, dont le centre coïncide sensiblement avec l'axe longitudinal défini par au moins la partie centrale précitée en double hélice.

6. Dispositif selon l'une des revendications 1 à 5, **caractérisé en ce que** le bord proximal (C) et le bord distal (D) sont compris dans un plan de coupe circulaire sensiblement perpendiculaire à l'axe longitudinal défini par au moins la partie centrale précitée en double hélice.

7. Dispositif selon l'une des revendications 1 à 5, **caractérisé en ce que** le bord proximal (C) et le bord distal (D) sont compris dans un plan de coupe circulaire selon un angle compris entre moins de 90° et au moins 60°, avantageusement entre moins de 90° et au moins 70°.

8. Dispositif selon l'une des revendications précédentes, **caractérisé en ce que** la double hélice étant composée de révolutions (R₁ à R₆) successives de 360°, au moins certaines révolutions comprennent au moins un bras de liaison (40, 42, 44) entre le premier élément (20) et le deuxième élément (30), avantageusement, chaque révolution de 360° comprend au moins un bras de liaison (40, 42, 44).

9. Dispositif selon la revendication 8, **caractérisé en ce que** chaque révolution (R₁ à R₆) comprend au moins deux bras de liaison (40, 42, 44).

10. Dispositif selon l'une des revendications précédentes, **caractérisé en ce que** la partie centrale en double hélice comprend au moins 3 coudes (CO) définissant au moins 3 sommets par révolution de 360°.

11. Dispositif selon l'une des revendications précédentes, **caractérisé en ce que** les parties successives de chaque élément (20, 30) définissant un coude (CO) sont de longueurs différentes et sont avantageusement égales deux à deux.

12. Dispositif selon l'une des revendications précédentes, **caractérisé en ce que** certains bras de liaison (40, 42, 44) ou chaque bras de liaison (40, 42, 44) est lui-même coudé en présentant un ou plusieurs coudes (CO) de manière à améliorer la flexibilité permettant d'améliorer aussi la conformabilité au conduit.

13. Dispositif selon l'une des revendications 1 à 12, **caractérisé en ce que** les bras de liaison (40, 42, 44) précités présentent une dimension suffisante pour permettre de définir un espace suffisant entre le premier (20) et le deuxième (30) éléments hélicoïdaux précités, pour le passage d'un outil dans un conduit adjacent au conduit dans lequel le dispositif formant endoprothèse doit être disposé.

14. Dispositif selon l'une des revendications 1 à 13, **caractérisé en ce que** la longueur du bras de liaison (40, 42, 44) est de l'ordre d'au moins 0,5 mm, avantageusement, la longueur du bras de liaison est comprise entre environ 0,5 mm et 5 mm.

15. Dispositif selon l'une des revendications précédentes, **caractérisé en ce que** la partie centrale comprend au moins un lien de renforcement (r) reliant deux sommets CO de deux révolutions d'hélicoïde, qui se suivent ; avantageusement, un ou plusieurs liens de renforcement sont présents par révolution, plus avantageusement réalisés en quinconce par rapport à des bras de liaison (40, 42, 44).

16. Dispositif selon l'une des revendications précédentes, **caractérisé en ce que** la section droite d'au moins l'un ou des deux dits premier (20) et deuxième (30) éléments filamentaire précités est choisie parmi le groupe consistant d'une section droite circulaire, d'une section droite polygonale telle que carré, rectangulaire, pentagonale, hexagonale ou octogonale, d'une section droite polygonale à bord arrondi, telle que carré à bord arrondi ou rectangulaire à bord arrondi, ou d'une section droite ovoïde.

17. Dispositif selon la revendication 16, **caractérisé en ce que** la dimension de la section droite est directement liée à la dimension de la section droite du conduit dans lequel le dispositif formant endoprothèse doit être inséré, généralement, le diamètre du premier élément ou du deuxième élément, ou des deux, est compris entre environ un centième et environ un dixième du diamètre du conduit dans lequel le dispositif formant endoprothèse doit être inséré.

18. Dispositif selon l'une des revendications 2 à 17, **caractérisé en ce que** le diamètre du bras de liaison (40, 42, 44) est sensiblement égal du diamètre de l'élément filamentaire, et avantageusement la forme du bras de liaison est aussi sensiblement identique à celle de l'élément filamentaire.

19. Dispositif selon l'une des revendications précédentes, **caractérisé en ce que** ledit premier élément (20) filamentaire ou le deuxième élément (30) filamentaire, ou les deux, définissent une hélicoïde présentant une inclinaison par rapport à l'axe longitudinal, au moins dans la partie centrale précitée, comprise entre environ 45° et environ 80°, avantageusement entre environ 50° et 70°, et encore mieux d'environ 60°.

20. Dispositif selon l'une des revendications 10 à 19, **caractérisé en ce que** chaque coude (CO) de l'élément filamentaire précité présente un angle aigu défini par deux parties successives de l'élément filamentaire, compris entre environ 20° et environ 60°.

21. Procédé de fabrication du dispositif formant endoprothèse, tel que défini à l'une quelconque des revendications 1 à 20, **caractérisé en ce qu'**on fabrique tout d'abord ledit premier élément (20) filamentaire précité de forme hélicoïdale et un deuxième élément (30) indépendant filamentaire de forme hélicoïdale, puis on relie lesdits premier élément et deuxième élément selon un pas déterminé, à des parties d'extrémités définissant un bord proximal et un bord distal, avec la présence de bras de liaison espacés le long de ladite partie centrale.

## Patentansprüche

1. Eine Endoprothese ausbildende Vorrichtung, welche ein erstes Fadenelement (20), das wenigstens in seinem mittleren Abschnitt schraubenförmig aufgewickelt ist, sowie einen proximalen Endabschnitt und einen distalen Endabschnitt umfaßt, wobei wenigstens ein Endabschnitt mit dem im wesentlichen ringförmig aufgewickelten Element ausgeführt ist,
**dadurch gekennzeichnet,**
**daß** die Vorrichtung ein zweites wenigstens in seinem mittleren Abschnitt schraubenförmig aufgewickeltes Fadenelement (30) aufweist, wobei das erste und das zweite Element somit eine Doppelschraubenstruktur definieren; und dadurch,
**daß** das erste Fadenelement (20) und das zweite Fadenelement (30) miteinander über wenigstens einige Verbindungsarme (40, 42, 44) verbunden sind, welche entlang des mittleren Abschnitts (16) angeordnet sind.

2. Vorrichtung nach Anspruch 1, **dadurch gekennzeichnet, daß** das erste Fadenelement (20) und das zweite Fadenelement (30), welche die zuvor erwähnte Doppelschraubenstruktur ausbilden, so gekrümmt sind, daß sie eine Zickzack-Form aufweisen, wobei sie hier eine schraubenförmige Zickzack-Form aufweisen.

3. Vorrichtung nach einem der vorhergehenden Ansprüche, **dadurch gekennzeichnet, daß** sie Endabschnitte (12, 14) umfaßt, welche jeweils einen proximalen Endrand (C) und einen distalen Endrand (D) der Vorrichtung definieren, wobei die Endabschnitte (12, 14) mit dem ersten und dem zweiten im wesentlichen ringförmig aufgewickelten Fadenelement ausgeführt und mit dem mittleren Abschnitt (16) verbunden sind, der aus dem zuvor genannten ersten doppelschraubenförmigen Fadenelement (20) und dem zweiten zuvor genannten doppelschraubenförmigen Fadenelement (30) besteht.

4. Vorrichtung nach einem der Ansprüche 1 bis 3, **dadurch gekennzeichnet, daß** die Endabschnitte (12, 14) eine allgemeine ringförmige Form aufweisen, welche direkt oder über eine Verbindung (32, 34) mit dem schraubenförmigen mittleren Abschnitt (16) verbunden ist.

5. Vorrichtung nach einem der Ansprüche 1 bis 4, **dadurch gekennzeichnet, daß** der proximale Endrand (C) und der distale Endrand (D) in einer kreisförmigen Schnittebene enthalten sind, deren Mittelpunkt im wesentlichen mit der Längsachse zusammenfällt, die durch wenigstens den zuvor genannten doppelschraubenförmigen mittleren Abschnitt definiert wird.

6. Vorrichtung nach einem der Ansprüche 1 bis 5, **dadurch gekennzeichnet, das** der proximale Rand (C) und der distale Rand (D) in einer kreisförmigen Schnittebene enthalten sind, welche im wesentlichen senkrecht zur Längsachse ist, welche durch wenigstens den zuvor genannten doppelschraubenförmigen mittleren Abschnitt definiert wird.

7. Vorrichtung nach einem der Ansprüche 1 bis 5, **dadurch gekennzeichnet, daß** der proximale Rand (C) und der distale Rand (D) in einer kreisförmigen Schnittebene entlang eines Winkels enthalten sind, der zwischen weniger als 90° und wenigstens 60°, vorteilhafterweise zwischen weniger als 90° und wenigstens 70° liegt.

8. Vorrichtung nach einem der vorhergehenden Ansprüche, **dadurch gekennzeichnet, daß** die Doppelschraube aus aufeinanderfolgenden Umläufen (R₁ bis R₆) von 360° besteht, wobei wenigstens bestimmte Umläufe wenigstens einen Verbindungsarm (40, 42, 44) zwischen dem ersten Element (20) und dem zweiten Element (30) umfassen, wobei vorteilhafterweise jeder 360°-Umlauf wenigstens einen Verbindungsarm (40, 42, 44) umfaßt.

9. Vorrichtung nach Anspruch 8, **dadurch gekennzeichnet, daß** jeder Umlauf (R₁ bis R₆) wenigstens zwei Verbindungsarme (40, 42, 44) umfaßt.

10. Vorrichtung nach einem der vorhergehenden Ansprüche, **dadurch gekennzeichnet, daß** der doppelschraubenförmige mittlere Abschnitt wenigstens 3 Krümmungen (CO) umfaßt, welche wenigstens 3 Spitzen pro 360°-Umlauf aufweisen.

11. Vorrichtung nach einem der vorhergehenden Ansprüche, **dadurch gekennzeichnet, daß** die aufeinanderfolgenden Abschnitte jedes Elementes (20, 30), welche eine Krümmung (CO) definieren, unterschiedliche Längen aufweisen und vorteilhafterweise paarweise gleich sind.

12. Vorrichtung nach einem der vorhergehenden Ansprüche, **dadurch gekennzeichnet, daß** bestimmte Verbindungsarme (40, 42, 44) oder jeder Verbindungsarm (40, 42, 44) selbst gekrümmt sind/ist, wobei sie/er eine oder mehrere Krümmungen (CO) aufweisen/aufweist, so daß die Biegsamkeit verbessert wird, wodurch ermöglicht wird, auch die Anpassungsfähigkeit an die Leitung zu verbessern.

13. Vorrichtung nach einem der Ansprüche 1 bis 12, **dadurch gekennzeichnet, daß** die Verbindungsarme (40, 42, 44) eine ausreichende Abmessung aufweisen, um die Definition eines Raumes zu ermöglichen, der zwischen dem ersten schraubenförmigen Element (20) und dem zweiten schraubenförmigen Element (30) für den Durchgang eines Werkzeugs in einer Leitung ausreichend ist, welche zu jener Leitung benachbart ist, in der die Endoprothese ausbildende Vorrichtung angeordnet werden muß.

14. Vorrichtung nach einem der Ansprüche 1 bis 13, **dadurch gekennzeichnet, daß** die Länge des Verbindungsarmes (40, 42, 44) in der Größenordnung von wenigstens 0,5 mm liegt, wobei die Länge des Verbindungsarmes vorteilhafterweise zwischen ungefähr 0,5 mm und 5 mm liegt.

15. Vorrichtung nach einem der vorhergehenden Ansprüche, **dadurch gekennzeichnet, daß** der mittlere Abschnitt wenigstens eine Verstärkungsverbindung (r) umfaßt, welche zwei Spitzen CO von zwei schraubenförmigen Umläufen, die aufeinander folgen, verbindet; vorteilhafterweise sind pro Umlauf eine oder mehrere Verstärkungsverbindungen vorhanden, noch vorteilhafterweise in Zickzackanordnung in Bezug auf die Verbindungsarme (40, 42, 44) ausgeführt.

16. Vorrichtung nach einem der vorhergehenden Ansprüche, **dadurch gekennzeichnet, daß** der gerade Abschnitt von wenigstens einem oder den zwei der genannten ersten (20) und zweiten (30) Fadenelemente aus der Gruppe ausgewählt ist, welche aus einem kreisförmigen Querschnitt, einem vieleckigen Querschnitt wie einem Quadrat, Rechteck, Fünfeck, Sechseck oder Achteck, einem vieleckigen Querschnitt mit abgerundetem Rand wie einem Quadrat mit abgerundetem Rand oder einem Rechteck mit abgerundetem Rand, oder einem eiförmigen Querschnitt besteht.

17. Vorrichtung nach Anspruch 16, **dadurch gekennzeichnet, daß** die Abmessung des Querschnitts direkt mit dem Abschnitt des Querschnitts der Leitung verbunden ist, in der die Endoprothese ausbildende Vorrichtung eingefügt werden muß, wobei im allgemeinen der Durchmesser des ersten Elementes oder des zweiten Elementes oder der zwei zwischen ungefähr einem Hundertstel und ungefähr einem Zehntel des Durchmessers der Leitung liegt, in der die Endoprothese ausbildende Vorrichtung eingefügt werden muß.

18. Vorrichtung nach einem der Ansprüche 2 bis 17, **dadurch gekennzeichnet, daß** der Durchmesser des Verbindungsarmes (40, 42, 44) im wesentlichen gleich dem Durchmesser des Fadenelementes ist, und vorteilhafterweise ist die Form des Verbindungsarmes auch im wesentlichen mit jener des Fadenelementes identisch.

19. Vorrichtung nach einem der vorhergehenden Ansprüche, **dadurch gekennzeichnet, daß** das erste Fadenelement (20) und das zweite Fadenelement (30), oder beide, eine Schraubenform definieren, weiche in Bezug auf die Längsachse, wenigstens im zuvor genannten mittleren Abschnitt, eine Neigung aufweist, die zwischen ungefähr 45° und ungefähr 80° liegt, vorteilhafterweise zwischen 50° und 70° und noch besser von ungefähr 60° ist.

20. Vorrichtung nach einem der Ansprüche 10 bis 19, **dadurch gekennzeichnet, daß** jede Krümmung (CO) des Fadenelementes einen spitzen Winkel aufweist, der durch zwei aufeinanderfolgende Abschnitte des Fadenelementes definiert ist und zwischen ungefähr 20° und ungefähr 60° liegt.

21. Verfahren zur Herstellung der Endoprothese ausbildenden Vorrichtung nach einem der Ansprüche 1 bis 20, **dadurch gekennzeichnet, daß** zunächst das erste schraubenförmige Fadenelement (20) und ein zweites schraubenförmiges unabhängiges Fadenelement (30) hergestellt wird, danach das erste und das zweite Element gemäß eines bestimmten Schritts mit Endabschnitten verbunden werden, welche einen proximalen Rand und einen distalen Rand definieren, wobei entlang des mittleren Abschnitts Verbindungsarme angeordnet sind.

## Claims

1. A stent forming device comprising a first filamentary element (20) which is spirally coiled at least in its central portion, and a proximal end portion and a distal end portion wherein at least one end portion is produced with said filamentary element coiled in substantially ring-like fashion, **characterised in that** it comprises a second filamentary element (30) which is spirally coiled at least in its central portion, said first and second elements thus defining a double helix structure ; and said first filamentary element (20) and said second filamentary element (30) are joined together by at least some linking arms (40, 42, 44) spaced out along said central portion (16).

2. The device according to claim 1, **characterised in that** said first element (20) and said second filamentary element (30) forming a double helix structure are bent so as to produce a zigzag form, in producing here a spiral zigzag form.

3. The device according to one of the preceding claims, **characterised in that** it comprises end portions (12, 14) which respectively define a proximal end edge (C) and distal end edge (D) of the device, said end portions (12, 14) being produced with said first and second filamentary elements coiled in substantially ring-like fashion and joined onto said central portion (16) composed of the first element (20) and of the second filamentary element (30) in a double helix.

4. The device according to one of claims 1 to 3, **characterised in that** the end portions (12, 14) produce a general ring-like form which is joined directly or via a joint (32, 34) to the double helix central portion (16).

5. The device according to one of claims 1 to 4, **characterised in that** the proximal edge (C) and the distal edge (D) of the ends are comprised in a plane of circular cross-section, the centre of which substantially coincides with the longitudinal axis which is defined by at least the double helix central portion.

6. The device according to one of claims 1 to 5, **characterised in that** the proximal edge (C) and the distal edge (D) are comprised in a plane of circular cross-section which is substantially perpendicular to the longitudinal axis which is defined by at least the central portion mentioned above in a double helix.

7. The device according to one of claims 1 to 5, **characterised in that** the proximal edge (C) and the distal edge (D) are comprised in a plane of circular cross-section according to an angle of between less than 90° and at least 60°, preferably between less than 90° and at least 70°.

8. The device according to one of the preceding claims, **characterised in that** the double helix being composed of successive turns (R₁ to R₆) of 360°, at least some turns comprise at least one linking arm (40, 42, 44) between the first element (20) and the second element (30), advantageously, each 360° turn comprises at least one linking arm (40, 42, 44).

9. The device according to claim 8, **characterised in that** each turn (R₁ to R₆) comprises at least two linking arms (40, 42, 44).

10. The device according to one of the preceding claims, **characterised in that** the central portion in a double helix comprises at least 3 bends (CO) defining at least 3 vertices per 360° turn.

11. The device according to one of the preceding claims, **characterised in that** the successive portions of each element (20, 30) defining a bend (CO) are of various lengths and are advantageously equal two by two.

12. The device according to any of the preceding claims, **characterized in that** some linking arms (40, 42, 44) or each linking arm (40, 42, 44) is itself bent with one or several bends (CO) so as to improve the flexibility enabling to also improve the conformability to the canal.

13. The device according to one of claims 1 to 12, **characterised in that** the linking arms (40, 42, 44) have a size which is sufficient to define a sufficient space between the first (20) and the second (30) spiral elements, for the passage of a tool in an adjacent canal to the canal in which the stent forming device must be disposed.

14. The device according to one of claims 1 to 13, **characterised in that** the length of the linking arm (40, 42, 44) is of the order of at least 0.5 mm, advantageously, the length of the linking arms is ranging between about 0.5 mm and 5 mm.

15. The device according to one of the preceding claims, **characterised in that** the central portion comprises at least one reinforcing joint (*r*) which joins two vertices CO of two successive spiral turns ; advantageously, one or more reinforcing joints are present per turn, which are more advantageously joined in a staggered manner with respect to the linking arms (40, 42, 44).

16. The device according to one of the preceding claims, **characterised in that** the straight cross-section of at least one or both said first (20) and second (30) filamentary elements is selected from the group consisting of a straight circular section, of a straight polygonal cross-section such as square, rectangular, pentagonal, hexagonal or octagonal, of a straight polygonal cross-section having rounded edges, such as square having rounded edges or rectangular having rounded edges, or of a straight oval cross-section.

17. The device according to claim 16, **characterised in that** the size of the straight cross-section is related directly to the size of the straight cross-section of the canal in which the stent forming device must be inserted, generally, the diameter of the first element or of the second element, or of both, is of between about one hundredth and about one tenth of the diameter of the canal in which the stent forming device must be inserted.

18. The device according to one of claims 2 to 17, **characterised in that** the diameter of the linking arm (40, 42, 44) is substantially equal to the diameter of the filamentary element, and advantageously the shape of the linking arm is also substantially identical to that of the filamentary element.

19. The device according to one of the preceding claims, **characterised in that** said first filamentary element (20) or the second filamentary element (30), or both, define a spiral having an inclination with respect to the longitudinal axis, at least in the central portion mentioned above, of between about 45° and about 80°, advantageously of between about 50° and 70°, and currently preferably of about 60°.

20. The device according to one of claims 12 to 19, **characterised in that** each bend (CO) of the filamentary element has an acute angle which is defined by two successive portions of the filamentary element, of between about 20° and about 60°.

21. A method of manufacture of the stent forming device, as defined in any one of claims 1 to 20, **characterised in that** it is manufactured, first of all, said first filamentary element (20) of spiral shape and a second independent filamentary element (30) of spiral shape, and then said first element and second element are joined, according to a determined pitch, to end portions defining a proximal edge and a distal edge, with the presence of linking arms spaced apart along said central portion.
